# FASCICULE DE BREVET EUROPEEN

(11) **EP 0 577 903 B1**
(45) Date de publication et mention de la délivrance du brevet: **17.12.1997**
(21) Numéro de dépôt: 92810515.4
(22) Date de dépôt: 06.07.1992
(51) Int. Cl.: A61K 35/74, C12N 1/20

(54) **Agent antigastrite**
Lactobacillus Acidophilus enthaltende Antigastritis-Mittel
Agent antigastrite contenant lactobacillus acidophilus

(43) Date de publication de la demande: 12.01.1994
(73) Titulaire: SOCIETE DES PRODUITS NESTLE S.A., 1800 Vevey (CH)
(72) Inventeur: Brassart, Dominique, CH-1034 Bussigny (CH); Michetti, Pierre, Division de Gastro-entérologie, CH-1011 Lausanne (CH); Neeser, Jean-Richard, CH-1010 Lausanne (CH)
(74) Mandataire: Wavre, Claude-Alain

(56) Documents cités:
- EP-A- 0 199 535
- WO-A-89/05849
- WO-A-91/09608
- Int. J. Syst. Bact., vol. 42, no.3, 487-791, 1992, Fujisawa et al.
- J. General Micro., vol.139, 513-517, 1993, Pot et al.

## Description

La présente invention a pour objet un agent antigastrite et/ou antiulcère, et une souche de bactérie lactique capable de produire cet agent.

On sait que certaines souches de bactéries lactiques présentent de bonnes facultés d'adhésion à des cellules intestinales et se prêtent de ce fait à des utilisations thérapeutiques.

EP 199535 (Gorbach et Goldin), par exemple, propose une souche de Lactobacillus dénommée GG du nom de ses inventeurs, et déposée à l'ATCC (American Type Culture Collection) sous le No 53103, qui est destinée à être administrée à l'homme ou à des animaux dans un but thérapeutique ou prophylactique au niveau du tractus digestif.

La présente invention a pour but de proposer un agent antigastrite et/ou antiulcère, et/ou une souche de bactérie lactique capable de produire cet agent, qui puissent être administrés à l'homme ou à des animaux dans un but thérapeutique ou prophylactique au niveau de l'estomac, en particulier au niveau du pylore.

A cet effet, la présente invention propose un agent antigastrite et/ou antiulcère présentant un pouvoir de déplacement de bactéries pathogènes sur des cellules intestinales et/ou gastriques, une culture biologiquement pure d'une souche de bactérie lactique sélectionnée pour sa faculté de produire un tel agent, et une composition comprenant d'une part un tel agent ou une telle culture en quantité efficace et d'autre part un support ingérable, en particulier un support pharmaceutiquement acceptable et/ou un produit alimentaire tel qu'un lait acidifié, notamment un yogourt ou une formule lactée en poudre.

On a constaté en effet que certaines souches de bactéries lactiques sont capables de déplacer des bactéries pathogènes telles que Helicobacter (H.) pylori, par exemple, sur des cellules intestinales auxquelles elles adhèrent. On a constaté également que ces souches présentent la faculté de produire un agent présentant un tel pouvoir de déplacement, et notamment de le produire dans leur milieu de culture.

L'agent, la souche ou la composition selon la présente invention sont donc tout particulièrement destinés à être administrés à l'homme ou à des animaux dans un but thérapeutique ou prophylactique au niveau de l'estomac, notamment dans le traitement de gastrites ou d'ulcères de l'estomac ou du pylore.

Parmi diverses souches sélectionnées selon la présente invention, l'une a été déposée, à titre d'exemple, selon le traité de Budapest, le 30.06.92, à la Collection Nationale de Cultures de Microorganismes (CNCM), Institut Pasteur, 28 rue du Dr Roux, 75724 Paris Cedex 15, France, où elle a reçu le No CNCM I-1225.

Des détails concernant la morphologie et les propriétés générales de cette souche sont donnés ci-après:

### Morphologie

- Microorganisme Gram-positif, non motile, ne formant pas de spores.
- Bâtonnets isolés assez courts et trapus

### Métabolisme

- Microorganisme microaérophile, avec métabolisme homofermentaire donnant lieu à la production d'acide lactique L(+) et D(-).
- Autres caractéristiques: Catalase (-), production de CO₂ (-), hydrolyse de l'arginine (-).

### Fermentation des sucres:

Amygdaline (+), arabinose (-), cellobiose (+), esculine (+), fructose (+),galactose (-), glucose (+), lactose (+), maltose (+/-), mannitol (-), mannose (+), melibiose (-), raffinose (+), ribose (-), salicine (+), sucrose (+), trehalose (+).

Des détails concernant les facultés particulières pour lesquelles la présente souche peut être sélectionnée sont donnés ci-après:

### Adhésion

Une adhésion à des cellules gastriques peut être assimilée à une adhésion à des cellules intestinales dans la mesure où les récepteurs reconnus par un microorganisme sur les deux types de cellules sont similaires et ce microorganisme présente une faculté de forte adhésion aux deux types de cellules.

Certains microorganismes pathogènes tels que Helicobacter pylori, par exemple, semblent posséder cette faculté.

On peut vérifier en particulier que Helicobacter pylori est capable d'adhérer à des cellules intestinales humaines dérivées d'adénocarcinomes, les cellules HT29 (Pinto et al., Biol.Cell.44, 193-196, 1982), par exemple, en culture monocouche in vitro.

Pour ce faire, on cultive les cellules HT29 à 37°C dans un milieu essentiel minimum Eagle modifié par Dulbecco (DMEM) contenant 10% de galactose et du sérum de veau dialysé, sous atmosphère à 10% de CO² et 90% d'air, et on les utilise avant le 20ème passage de culture. On réalise les cultures sur des lamelles de verre dégraissées placées dans des boîtes à 24 puits.

On cultive H.pylori sur plaques Müller-Hinton 10% sang de mouton, à 37°C sous atmosphère à 5% d'O₂, 10% de CO₂ et 85% d'azote, durant 72 h. On gratte les plaques, on récolte les bactéries et on les lave en solution physiologique.

On inocule H.pylori sur les cellules HT29, à raison de 10⁶ germes viables ou cellules (cfu) par cm². On incube 2h à 37°C et on lave trois fois les monocouches.

On détermine le nombre de cellules de H.pylori qui ont adhéré aux cellules HT29 à l'aide d'un test uréase (Jatrox-Hp-Test) dont le principe est qu'une solution aqueuse d'urée et de rouge phénol passe du jaune au rose fuchsia en présence d'uréase, qui catabolise la production de métabolites basiques de l'urée, l'ammonium et le bicarbonate. L'intensité de la réaction est lue au spectrophotomètre à 550nm. Ce test est linéaire pour des valeurs comprises entre 10⁴ et 10⁶ cfu/ml.

### Déplacement

Pour déterminer un pouvoir de déplacement de bactéries pathogènes présenté par une souche, une culture, un agent et ou une composition selon la présente invention, on peut examiner dans quelle mesure ils sont capables de déplacer les H.pylori adhérents aux cellules HT29, par exemple.

Pour ce faire, on peut ajouter une solution ou suspension de bactérie lactique à sélectionner ou d'agent à tester aux cellules HT29 auxquelles adhèrent les cellules de H.pylori, incuber durant 1h, laver plusieurs fois et pratiquer le test uréase.

Si l'on ajoute ainsi une culture de la souche L.acidophilus CNCM I-1225, par exemple, avec son milieu de culture (MRS ou lait, par exemple) dilué à 50% dans du DMEM, à raison de 10⁷ cfu par cm², on constate que des 2.10⁶ cfu de H.pylori adhérentes dénombrées par cm² en l'absence de bactérie compétitrice, il ne reste que 6.10³ cfu/cm² après incubation de 1h avec cette souche L.acidophilus CNCM I-1225.

Ceci montre tout l'intérêt que peut présenter une telle souche dans le traitement de gastrites ou d'ulcères de l'estomac ou du pylore.

De même, si l'on ajoute aux cellules HT29 auxquelles adhèrent les cellules de H.pylori le surnageant de la culture de L.acidophilus ci-dessus, on observe également un très fort déplacement de H.pylori.
Ceci montre que certaines souches de bactéries lactiques, en l'occurrence la souche L.acidophilus CNCM I-1225, peuvent sécréter un agent antigastrite et/ou antiulcère dans leur milieu de culture. Un tel surnageant et un tel agent extrait de ce surnageant peuvent donc eux-mêmes présenter un très grand intérêt dans le traitement de gastrites ou d'ulcères de l'estomac ou du pylore.

## Revendications

1. Utilisation d'une bactérie lactique présentant un pouvoir de déplacement d'*Helicobacter pylori* sur des cellules intestinales ou gastriques ou d'un surnageant de culture ou d'un extrait de culture d'une bactérie lactique ledit surnageant ou ledit extrait présentant un pouvoir de déplacement d'*Helicobacter pylori* sur des cellules intestinales ou gastriques dans la préparation d'un support destiné au traitement thérapeutique ou prophylactique de l'ulcère associé à l'action de *Helicobacter pylori*.

2. Utilisation selon la revendication 1, dans lequel le support est un support pharmaceutiquement acceptable ou un produit alimentaire.

3. Utilisation selon la revendication 1, d'un surnageant de culture ou d'un extrait de surnageant culture d'une bactérie lactique.

4. Utilisation selon l'une des revendication 1 à 3, de la souche de bactérie lactique CNCM I-1225.

5. Souche de bactérie lactique CNCM I-1225.

## Claims

1. Use of a lactic acid bacterium having the ability to displace *Helicobacter pylori* on intestinal or gastric cells or of a culture supernatant or of a culture extract of a lactic acid bacterium, the said supernatant or the said extract having the ability to displace *Helicobacter pylori* on intestinal or gastric cells, in the preparation of a support intended for the therapeutic or prophylactic treatment of an ulcer associated with the action of *Helicobacter pylori*.

2. Use according to claim 1, wherein the support is a pharmaceutically acceptable support or a food product.

3. Use according to claim 1 of a culture supernatant or of an extract of a culture supernatant of a lactic acid bacterium.

4. Use according to one of claims 1 to 3, of the lactic acid bacterial strain CNCM I-1225.

5. Lactic acid bacterial strain CNCM I-1225.

## Patentansprüche

1. Verwendung einer Milchsäurebakterie, die die Fähigkeit zur Verdrängung von Helicobacter pylori von Darm- und Magenzellen aufweist, oder eines Kulturüberstands oder eines Extrakts einer Kultur einer Milchsäurebakterie, wobei der genannte Überstand oder genannte Extrakt die Fähigkeit aufweist, Helicobacter pylori von Darm- oder Magenzellen zu verdrängen, zur Herstellung eines Trägers, der für die therapeutische oder prophylaktische Behandlung eines Magengeschwürs bestimmt ist, das auf die Wirkung von Helicobacter pylori zurückgeht.

2. Verwendung nach Anspruch 1, bei der der Träger ein pharmazeutisch akzeptabler Träger oder ein Nahrungsmittelprodukt ist.

3. Verwendung nach Anspruch 1 eines Kulturüberstands oder eines Extrakts eines Kulturüberstands einer Milchsäurebakterie.

4. Verwendung nach einem der Ansprüche 1 bis 3 des Milchsäurebakterien-Stammes CNCM I-1225.

5. Milchsäurebakterien-Stamm CNCM I-1225.
